Europäisches Patentamt

(19) European Patent Office

Office européen des brevets

(11) Numéro de publication: **0 116 251**
**B1**

(12) **FASCICULE DE BREVET EUROPÉEN**

(45) Date de publication du fascicule du brevet:
16.12.87

(21) Numéro de dépôt: **83402534.8**

(22) Date de dépôt: **26.12.83**

(51) Int. Cl.⁴: **C 08 B 5/14,** C 08 B 31/06,
C 08 B 37/00

(54) Procédé pour la dépolymérisation et la sulfatation de polysaccharides.

(30) Priorité: **28.12.82 FR 8221934**
**06.12.83 FR 8319506**

(43) Date de publication de la demande:
**22.08.84 Bulletin 84/34**

(45) Mention de la délivrance du brevet:
**16.12.87 Bulletin 87/51**

(84) Etats contractants désignés:
**AT BE CH DE FR GB IT LI LU NL SE**

(56) Documents cité:
**FR-A-1 093 999**
**US-A-2 025 073**
**US-A-2 755 275**
**US-A-3 454 560**
**US-A-3 498 972**

**Le dossier contient des informations techniques présentées postérieurement au dépôt de la demande et ne figurant pas dans le présent fascicule.**

(73) Titulaire: **SCLAVO S.p.A., Via Fiorentina 1, I-53100 Siena (IT)**
Titulaire: **SANOFI, société anonyme, 40, Avenue George V, F-75008 Paris (FR)**

(72) Inventeur: **Naggi, Annamaria, Via Monte Nevoso N. 32, Legnano Milano (IT)**
Inventeur: **Torri, Giangiacomo, Via Confalonieri 8, Bergamo (IT)**

(74) Mandataire: **Gillard, Marie- Louise, Cabinet Beau de Loménie 55, Rue d'Amsterdam, F-75008 Paris (FR)**

EP 0 116 251 B1

## Description

La présente invention concerne un procédé pour la dépolymérisation et la sulfatation de polysacharides.

Les polysaccharides sulfatés sont des composés ayant une très grande importance dans l'industrie cosmétique, textile, alimentaire et pharmaceutique. Notamment leur utilisation est préconisée dans la prévention de la thrombose veineuse (I.B. Jacques, Pharmacological Reviews 1979, 31 99-166).

En outre, les polysaccharides sulfatés de bas poids moléculaire ont été proposés comme agents antithrombotiques non anti-coagulants comportant donc un risque hémorragique faible (D.P. Thomas, Seminars in Hematology, 1978, 15, 1-17).

Les polysaccharides sulfatés de bas poids moléculaire sont obtenus par sulfatation de polysaccharides de bas poids moléculaire. Les polysaccharides de bas poids moléculaire sont généralement obtenus par fractionnement d'un ensemble d'espèces à poids moléculaire varié ou bien par dépolymérisation, contr*olée de polysaccharides non fractionnés à l'aide de l'acide nitreux.

La sulfatation est effectuée généralement par traitement avec de l'acide chlorosulfonique dans de la pyridine (M.L. Wolfrom et al., J. Am. Chem. Soc., 1953, 75, 1519) ou bien avec des produits d'addition du trioxyde de soufre (anhydride sulfurique) avec des solvants aprotiques (R.L. Whilster, W.W. Spencer, Methods Carborhydrate Chem., 1964, 4 297-296 ; R.L. Whilster, ibid., 1972, 6, 426-429).

A cet effet, on peut se référer également aux brevets US 2.025.073, 2.755.275, 3.498.972 et 3.454.560.

Le brevet US 2.755.275 décrit la sulfatation de la chitine, entre autres, par l'acide chlorosulfonique ou de trioxyde de soufre. Cette sulfatation est effectuée en présence d'un diluant, notamment de dichloroéthane.

Les brevets US 3.498.972 et 3.454.560 décrivent un procédé pour la sulfatation, respectivement, du dextran et du chondroitinsulfate par l'acide sulfurique sans utiliser des solvants organiques. Dans les deux brevets, il est indiqué que l'acide sulfurique peut contenir d'autres agents de sulfatation comme l'acide chlorosulfonique ou le trioxyde de soufre. Il est cependant ajouté que le vrai agent de sulfatation n'est que l'acide sulfurique.

Le brevet US 2.025.073 concerne la préparation de dérivés cellulosiques par traitement de la cellulose avec de l'aide pyrosulfurique en présence d'une amine tertiaire, telle que la pyridine avec ou sans diluant.

Les procédés de sulfatation connus présentent cependant certains inconvénients dus notamment aux conditions opératoires et à la difficulté de contr*oler la réaction.

Les procédés de dépolymérisation, de leur c*oté, présentent le désavantage de donner certains pourcentages de produits inactifs.

Dans le cas de polysaccharides N-sulfatés, tels que l'héparine, les procédés de dépolymérisation comportent également une hydrolyse desdits groupes N-sulfatés, indispensables pour l'activité biologique de l'héparine.

On a maintenant trouvé d'une façon surprenante que, par réaction d'un polysaccharide avec uniquement un mélange d'acide sulfurique et d'acide chlorosulfonique, on obtient à la fois une dépolymérisation et une sulfatation. Ce fait est particulièrement surprenant, d'autant plus qu'on a également trouvé que la sulfatation est toujours totale sur les hydroxyles primaires éventuellement présents.

Ainsi, la présente invention a pour objet un procédé pour la dépolymérisation et la sulfatation des polysaccharides autres que l'héparine, caractérisé, en ce qu'on traite un polysaccharide avec uniquement un mélange d'acide sulfurique et d'acide chlorosulfonique.

Les deux acides dans le mélange sont concentrés et, de préférence, leur concentration est au moins supérieure à 95 % en poids.

Le rapport des deux acides est très variable et peut aller de traces d'acide chlorosulfonique dans l'acide sulfurique jusqu'à un rapport acide sulfurique:acide chlorosulfonique 1:4 en volume. De préférence, le rapport acide sulfurique:acide chlorosulfonique varie de 4:1 à 1:1, un rapport d'environ 2:1 étant particulièrement préféré.

La température de réaction et la concentration de produit de départ dans le mélange acide sulfurique/acide chlorosulfonique peuvent varier selon la nature du substrat. Par exemple, la faible solubilité de la cellulose suggère des dilutions plus élevées, tandis que, dans le cas du chitosane, il est possible d'utiliser une concentration supérieure et d'effectuer la réaction à température relativement basse.

En général, la température de réaction peut varier entre -20 et +40°C ; après une période variable en raison de la température, de quelques minutes à 2 heures, la réaction est achevée et le polysaccharide dépolymérisé et sulfaté est isolé selon les techniques classiques, par exemple par neutralisation et dialyse, par chromatographie ou par lyophilisation.

On peut aussi isoler le polysaccharide dépolymérisé et sulfaté en versant le mélange réactionnel dans un solvant dans lequel le produit final est insoluble, par exemple un solvant apolaire aprotique, tel que l'éther diéthylique, en filtrant le précipité qui s'est formé et en le purifiant selon les techniques connues dans la chimie des sucres.

Les polysaccharides dépolymérisés et sulfatés peuvent être isolés sous forme de sels alcalins selon les techniques usuelles, par exemple par lyophilisation ou par évaporation sous pression réduite, et caractérisés par les méthodes physico-chimiques connues.

D'autres sels, par exemple le sel de calcium, peuvent être obtenus à partir des sels alcalins, le

sel de sodium de préférence, par réaction d'échange avec le sel approprié, par exemple un sel de calcium, en utilisant éventuellement une résine échangeuse d'ions.

Dans le cas des polysaccharides de départ ayant un degré de polymérisation très élevé, par example dans le cas du chitosane, de la chitine ou de la cellulose, il est souhaitable de soumettre d'abord ledit composé de départ à une première dépolymérisation selon les méthodes connues, par example par traitement avec de l'acide nitreux. Le produit ainsi préalablement partiellement dépolymérisé peut être ultérieurement dépolymérisé et sulfaté selon le procédé de la présente invention.

On peut aussi soumettre le polysaccharide de départ ayant un poids moléculaire très élevé, deux fois au procédé de la présente invention. Dans ce cas, il n'est même pas nécassaire d'isoler le produit dépolymérisé ; on peut ajouter une deuxième quantité du mélange acide sulfurique/acide chlorosulfonique après, par exemple, la première heure de réaction. Cette procédure, de façon surprenante, ne comporte pas de dégradation ni de sulfatation ultérieure. Par exemple, dans le cas de la cellulose, on obtient, par cette procédure, un composé dépolymérisé sélectivement sulfaté dans la position 6, à savoir sur l'hydroxyle primaire.

Le procédé de la présente invention peut être mis en oeuvre avec les polysaccharides connus. Des produits de départ convenables sont les héparansulfates, le chitosane, la chitine, la cellulose, l'amidon, le guarane, les chondroitinsulfates, les polyxylanes, l'inuline, le dermatansulfate, le kératane, les mannanes, le scléroglucane, les galactomannanes, les dextranes, les galactanes, le xantane.

L'avantage du procédé de la présente invention réside dans sa sélectivité et dans sa maniabilité.

Dans le cas du chitosane, par réaction avec un mélange acide sulfurique/acide chlorosulfonique selon la présente invention on obtient un chitosane dont on ne connaît pas le degré de dépolymérisation car son poids moléculaire, comme celui du composé de départ, est trop élevé, mais que l'on suppose être dépolymérisé. Ce composé a les hydroxyles primaires sélectivement sulfatés, sans qu'il y ait variation dans l'hydroxyle secondaire et le groupe amino libre.

En outre, selon le procédé de la présente invention il est possible de contr*oler le degré de sulfatation en faisant varier la température et/ou le temps de réaction de façon opportune. Par exemple, encore dans le cas du chitosane, il est possible d'obtenir un chitosane ayant un degré de sulfatation, toujours sélective en position 6, qui est supérieur à zéro et qui peut arriver à 1.

La cellulose, l'amidon, la chitine se comportent comme le chitosane.

Le chondroitinsulfate et le dermatansulfate se comportent comme l'héparine.

Dans le cas du guarane, on peut obtenir des guaranes dépolymérisés portant un groupe sulfate sur l'hydroxyle primaire du D-mannose.

Le degré de dépolymérisation varie selon le poids moléculaire du produit de départ et de la stabilité des liaisons glucosidiques. De ce point de vue, la chitine et le chitosane sont plus stables et la dépolymérisation est mineure.

Dans le cas de la cellulose et de l'amidon, on obtient des produits dépolymérisés et sulfatés ayant un degré de dépolymérisation plus élevé.

Le chondroitinsulfate et le dermatansulfate sont moins stables et la dépolymérisation peut arriver jusqu'à des tri- et tétrasaccharides.

En géneral, le degré de dépolymérisation peut être contr*olé en modifiant de façon opportune le rapport acide sulfurique/acide chlorosulfonique, le temps de réaction ainsi que la concentration du produit de départ dans le mélange des deux acides.

Les exemples suivants illustrent l'invention sans toutefois la limiter.

## Exemple 1

A un mélangé de 20 ml d'acide sulfurique à 95 % et 10 ml d'acide chlorosulfonique, préalablement refroidi à une température d'environ 0-4°C, on ajoute 500 mg de chitosane ANIC, lot 116, et on agite le mélange à la même température pendant environ 1 heure. On verse ensuite le mélange réactionnel dans de l'éther diéthylique préalablement refroidi, on filtre le précipité ainsi obtenu et on le neutralise avec une solution de bicarbonate de potassium. Après une dialyse dans des tubes à 8500 D, on obtient un chitosane 6-sulfate ayant les caractéristiques suivantes :
- Degré de substitution (par voie conductimétrique) : 1.
- Spectre IR : large bande dans la région 1300-1200 $cm^{-1}$, caractéristique des groupes sulfates.
- Spectre 13C-RMN : disparition du signal relatif au groupe hydroxyle primaire et apparition du signal relatif aux groupes sulfates.

## Exemple 2

A un mélange de 20 ml d'acide sulfurique à 95 % et 10 ml d'acide chlorosulfonique à 98 %, refroidi à une température entre -4 et 0°C, on ajoute 1 g de chitosane ANIC, lot 116. On laisse le mélange réactionnel 30 minutes à la température ambiante, puis on le verse dans 500 ml d'éther diéthylique préalablement refroidi. On filtre, on lave le précipité avec de l'éther diéthylique froid, on le dissout dans de l'eau et on le neutralise avec une solution d'hydroxyde de sodium 0,5 N. On dialyse contre de l'eau distillée dans des membranes à 8000 D (THOMAS DIALYZER TUBING) et l'on évapore sous pression réduite. On obtient ainsi, avec un rendement de 90 % en poids, un chitosane 6-sulfate ayant les caractéristiques suivantes :
- Degré de substitution (par voie conductimétrique) : 0,5 ; à savoir, seulement 50 % des groupes hydroxyles en position 6 ont été sulfatés.
- Spectre IR : large bande dans la région 1300-1200 cm$^{-1}$, caractéristique des groupes sulfates.
- Spectre 13C-RMN : diminution du signal relatif aux groupes hydroxyles primaires et apparition du signal relatif aux groupes sulfates.

## Example 3

a) A une solution de 1 g de chitosane ANIC, lot 116, dans 50 ml d'acide acétique à 30 %, on ajoute 2,3 ml d'acide nitreux 0,5 M préparé à partir de 10 ml de nitrite de baryum monohydraté 0,5 M et 10 ml d'acide sulfurique 0,5 M, puis on agite à la température ambiante pendant 12 heures, on concentre sous pression réduite et l'on précipite avec de l'acétone. On filtre le précipité, on le lave avec de l'acétone, on le sèche, on le dissout dans de l'eau et on le traite avec 30 mg de borohydrure de sodium. Après 12 heures à la température ambiante on détruit l'excès de borohydrure de sodium avec de "l'Amberlite IR 120 H +" et on élimine l'acide borique par évaporation sous pression réduite en présence de méthanol. On obtient ainsi un chitosane dépolymérisé ayant un poids moléculaire très inférieur à celui du chitosane de départ.

b) A un mélange de 20 ml d'acide sulfurique à 95 % et de 10 ml d'acide chlorosulfonique à 98 %, refroidi à une température entre -4 et 0°C, on ajoute 1 g de chitosane dépolymérisé obtenu comme décrit ci-dessus. On laisse le mélange réactionnel à la même température pendant 1 heure, on le verse dans 250 ml d'éther diéthylique préalablement refroidi, on filtre le précipité et on le lave avec de l'éther diéthylique froid. On dissout le produit dans de l'eau et l'on neutralise avec une solution d'hydroxyde de sodium 0,5 M. Après élimination du sel par chromatographie sur "Séphadex G25", on obtient, avec un rendement de 90 %, un chitosane 6-sulfate dépolymérisé ayant les caractéristiques suivantes :
- Degré de substitution : 1.
- Spectre IR : large bande dans la région 1300-1200 cm$^{-1}$, caractéristique des groupes sulfates.
- Spectre 13C-RMN : disparition du signal relatif au groupe hydroxyle primaire et apparition d'un nouveau signal dû au groupe sulfate.

## Exemple 4

A un mélange de 20 ml d'acide sulfurique à 95 % et 10 ml d'acide chlorosulfonique à 98 %, refroidi à 0-4°C, on ajoute 1 g de cellulose microcristalline (P:M. : 20000). On laisse le mélange réactionnel sous agitation 1 heure à la température ambiante, puis on ajoute encore 30 ml du mélange acide sulfurique:acide chlorosulfonique 2:1. Après 30 minutes, on verse le mélange dans 500 ml d'éther diéthylique froid, on filtre, on lave le précipité avec de l'éther diéthylique et on le dissout dans de l'eau. Par neutralisation avec une solution d'hydroxyde de sodium 0,5 M, dialyse dans des tubes à 3500 D (THOMAS DIALYZER TUBING 3787-H47, diamètre de 11 mm) et évaporation sous pression réduite, on obtient, avec un rendement de 36 % de la fraction dialysable et de 29 % de la fraction non dialysable, une cellulose 6-sulfate ayant les caractéristiques suivantes :
- Degré de substitution (par voie conductimétrique) : 1.
- Spectre IR : large bande dans la région 1300-1200 cm$^{-1}$, caractéristique des groupes sulfates.
- Poids moléculaire : 3400.

## Example 5

A un mélange de 10 ml d'acide sulfurique à 98 % et de 5 ml d'acide chlorosulfonique à 98 % à la température de 0-4°C, on ajoute 1 g de guarane (AGOGUM F-90, lot 433). Après 1 heure à la même température, on obtient un guarane 6-sulfate, isolé sous forme de sel de sodium (numéro de code : AH-102), ayant les caractéristiques suivantes :.
- Degré de substitution (par voie conductimétrique) : 1.
- Spectre IR : large pande dans la region 1300-1200 cm$^{-1}$ , caractéristique des groupes sulfates.
- Rendement : 26 % en poids.

## Exemple 6

A un mélange de 20 ml d'acide sulfurique à 98 % et 10 ml d'acide chlorosulfonique à 95 %, refroidi à 0-4°C, on ajoute 1 g de chitine SIGMA (lot 12 F-7060), puis on abandonne le mélange réactionnel 1 heure à la même température. En opérant ensuite comme décrit dans l'exemple 1, on obtiént, après dialyse et évaporation sous pression réduite, une chitine 6-sulfate, isolée sous forme de sel de sodium (numéro de code : AH-50) ayant les caractéristiques suivantes :
- Degré de substitution (par voie conductimétrique) : 1.
- Spectre IR : large bande dans la région 1300-1200 cm$^{-1}$, caractéristique des groupes sulfates.
- Spectre 13C-RMN : disparition du signal de l'hydroxyle primaire et apparition du signal des groupes sulfates.
- Rendement : 70 % en poids.

## Exemple 7

A 15 ml d'un mélange acide sulfurique à 95 % : acide chlorosulfonique à 98 % 2:1, refroidi à 0-4°C, on ajoute 500 mg de chondroitinsulfate TAKEDA (lot GB-185, numéro de code : D-267) ayant un poids moléculaire de 22000 et contenant environ 18 % d'humidité. Après 1 heure à la température ambiante, on verse le mélange dans 500 ml d'éther diéthylique froid, on dissout le précipité dans l'eau, on neutralise la solution ainsi obtenue avec de l'hydroxyde de sodium 0,5 M. On dialyse ensuite dans des tubes à 3500 D (THOMAS DIALYZER TUBING, diamètre 15 mm). Par évaporation sous pression réduite, on obtient un chondroitinsulfate dépolymérisé (numéro de code : AH-69) ayant les caractéristiques suivantes:

- Spectre IR : large bande dans la région 1300-1200$^{-1}$ cm, caractéristique des groupes sulfates.
- Poids moléculaire : 2000.

## Exemple 8

A un mélange de 10 ml d'acide sulfurique à 98 % et 5 ml d'acide chlorosulfonique à 98 %, on ajoute 500 ml de dermatansulfate OPOCRIN (lot 7-8 HF) ayant un poids moléculaire 27000 et un degré de substitution ($SO_3^-$/$COO^-$):1.

En opérant comme décrit dans l'exemple 25, on obtient un dermatansulfate dépolymérisé et supersulfaté (numéro de code : AH-79) ayant les caractéristiques suivantes :

- Degré de substitution ($SO_3^-$/$COO^-$, voie conductimétrique) : 2,8.
- Spectre IR : large bande dans la région 1300-1200 cm$^{-1}$, caractéristique des groupes sulfates.
- Poids moléculaires : 2000.

## Revendications

Procédé pour la dépolymérisation et la sulfatation des polysaccharides autres que l'héparine, caractérisé en ce qu'on fait réagir ledit polysaccharide avec uniquement un mélange d'acide sulfurique et d'acide chlorosulfonique.

2. Procédé selon la revendication 1, caractérisé en ce que la réaction est effectuée à une temperature de - 20 à +40°C.

3. Procédé selon l'une des revendications 1 ou 2, caractérisé en ce que la concentration des deux acides est d'au moins 95 % en poids.

4. Procédé selon l'une quelconque des revendications 1 à 3, caractérisé en ce que le rapport acide sulfurique: acide chlorosulfonique est compris entre 4:1 et 1:1.

5. Procédé selon l'une quelconque des revendications 1 à 4, caractérisé en ce que le rapport acide sulfurique : acide chlorosulfonique est environ 2:1.

6. Procédé selon l'une quelconque des revendications 1 à 5, caractérisé en ce que le polysaccharide dépolymérisé et sulfaté est isolé sous forme de sel de sodium.

7. Procédé selon l'une quelconque des revendications 1 à 6, caractérisé en ce que, comme produit de départ, on utilise un polysaccharide préalablement partiellement dépolymérisé.

## Patentansprüche

1. Verfahren für die Depolymerisation und die Sulfatation von anderen Polysacchariden als Heparin, dadurch gekennzeichnet, dass man das genannte Polysaccharid mit nur einer Mischung von Schwefelsäure und Chlorsulfonsäure reagieren lässt.

2. Verfahren nach Anspruch 1, dadurch gekennzeichnet, dass die Umsetzung bei einer Temperatur zwischen - 20 und + 40°C erfolgt.

3. Verfahren nach einem der Ansprüche 1 oder 2, dadurch gekennzeichnet, dass die Konzentration der beiden Säuren mindestens 95 Gew. % beträgt.

4. Verfahren nach einem der Ansprüche 1 bis 3, dadurch gekennzeichnet, dass das Verhältnis Schwefelsäure : Chlorsulfonsäure zwischen 4 : 1 und 1 : 1 liegt.

5. Verfahren nach einem der Ansprüche 1 bis 4, dadurch gekennzeichnet, dass das Verhältnis Schwefelsäure : Chlorsulfonsäure etwa 2 : 1 beträgt.

6. Verfahren nach einem der Ansprüche 1 bis 5, dadurch gekennzeichnet, dass das depolymerisierte und sulfatisierte Polysaccharid in Form des Natriumsalzes isoliert wird.

7. Verfahren nach einem der Ansprüche 1 bis 6, dadurch gekennzeichnet, dass man als Ausgangsprodukt ein zuvor teilweise depolymerisiertes Polysaccharid benutzt.

## Claims

1. A process for the depolymerisation and sulfatation of polysaccharides other than heparin, characterised in that said polysaccharide is reacted with only one mixture of sulfuric acid and chlorosulfonic acid.

2. A process according to claim 1, characterised in that the reaction is carried out at a temperature of from -20° to +40°C.

3. A process according to one of claims 1 or 2, characterised in that the concentration of the two acids is at least 95 % by weight.

4. A process according to any one of claims 1 to 3, characterised in that the ratio sulfuric acid : chlorosulfonic acid is from 4:1 to 1:1.

5. A process according to any one of claims 1 to 4, characterised in that the ratio sulfuric acid : chlorosulfonic acid is about 2 : 1.

6. A process according to any one of claims 1 to 5, characterised in that the depolymerised and sulfated polysaccharide is isolated in the form of sodium salt.

7. A process according to any of claims 1 to 6, characterised in that a polysaccharide previously partially depolymerized is used as starting product.